(19) Europäisches Patentamt
European Patent Office
Office européen des brevets

(11) **EP 4 600 857 A1**

(12) **EUROPEAN PATENT APPLICATION**

(43) Date of publication:
**13.08.2025 Bulletin 2025/33**

(21) Application number: **24164025.9**

(22) Date of filing: **18.03.2024**

(51) International Patent Classification (IPC):
***G06F 21/62*** *(2013.01)*

(52) Cooperative Patent Classification (CPC):
**G06F 21/6254; G16H 10/60; G16H 40/67**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **06.02.2024 PCT/CN2024/076423**

(71) Applicant: **Koninklijke Philips N.V.
5656 AG Eindhoven (NL)**

(72) Inventor: **ZHANG, Fengchang
Eindhoven (NL)**

(74) Representative: **Philips Intellectual Property &
Standards
High Tech Campus 52
5656 AG Eindhoven (NL)**

(54) **DETERMINATION OF DATA RISK**

(57) Example embodiments of the present disclosure relate to a method for determining a data risk, an electronic device, and a computer-readable medium. In the solution, a first data risk value is determined based on a re-identification probability of each record in the sample dataset, and a second data risk value is determined based on statistical information of a population which includes the sample dataset. In addition, a data risk may be determined based on the first data risk value and the second data risk value. Embodiments of the present disclosure consider both a re-identification probability of each record in the sample dataset and statistical information of a population for determining the data risk. Therefore, the determined data risk will be more accurate, thereby an accurate re-identification risk may be obtained accordingly.

**FIG. 1A**

Processed by Luminess, 75001 PARIS (FR)

**Description**

TECHNICAL FIELD

Embodiments of the present disclosure relate to a field of data processing, and more specifically, to a determination of data risk.

BACKGROUND

Personal data protection is an important issue in the security and privacy domain. Specifically, personal data should be de-identified for a second purpose usage, e.g., as required by some regulations like General Data Protection Regulation (GDPR) in Europe and Personal Information Protection Law in China. In the United States, the Health Insurance Portability and Accountability Act (HIPAA) specifies 18 specific categories of information that must be removed from medical records to be used in research.

Some de-identification techniques have been introduced for processing data to enable the exploitation of the benefits of data (such as the second purpose usage) while maintaining compliance with the relevant privacy regulations. After applying a de-identification approach based on the de-identification techniques, a quantified re-identification risk should be obtained. Quantifying the re-identification risk involves a determination of a data risk and a context risk. However, there are still some challenges in determining the data risk that need to be addressed in order to improve an accuracy of the re-identification risk.

SUMMARY

There are various methods to determine the data risk, e.g., depending on an attack type. Those methods are closely related to the type of attack assumed before determining a data risk. However, the current methods rely on a single attack type assumption, which means the data risk estimation is either based only on sample dataset or the statistical information of prevalent population. As a result, the accuracy of the determined data risk depends on the relationship between the sample dataset and the prevalent population (e.g., whether the sample dataset can represent the population accurately) and thus may not be right.

In view of the above, embodiments of the present disclosure provide a method for determining a data risk based on the sample dataset, an electronic device, and a computer-readable medium. The embodiments of the present disclosure can determine a data risk by consolidating the possibilities of different attack types. Thus, it is not necessary to assume the attack types before determining the data risk.

In a first aspect, the embodiments of the present disclosure provide a method for determining a data risk. The sample dataset comprises a plurality of records, and the method comprises: determining a first data risk value based on a re-identification probability of each record in the plurality of records in the sample dataset; determining a second data risk value based on statistical information of a population, wherein the population comprises the sample dataset; and processing at least the first data risk value and the second data risk value to determine the data risk.

According to the embodiments of the present disclosure, the method may determine a data risk by considering both a re-identification probability of each record in the sample dataset and statistical information of a population which includes the sample dataset. Data risk is a kind of risk with respect to a subject in a population, and the subject may or may not be included in the sample dataset. Data risk refers to the risks arising from inadequate data security controls, for example, in the course of operations. Data risks include, but are not limited to, data leakage, data corruption, data inconsistency, and other issues. The subject can be the owner of one record, can be the record itself, or can be the information included in the record. In this invention, both sample dataset level information and population level information are used to determine the data risk. Therefore, the determined data risk will be more accurate; thereby an accurate re-identification risk may be obtained accordingly.

In some embodiments of the first aspect, the method further comprises determining whether or not a data recipient of the sample dataset has information about a probability that a target record is included in the sample dataset. In this way, whether the data recipient has information about a probability that a target record is included in the sample dataset may be further considered. Thus, information used for determining the data risk will be more comprehensive to improve the accuracy of the determined data risk.

In some embodiments of the first aspect, processing at least the first data risk value and the second data risk value to determine the data risk comprises: responsive to a determination that the data recipient does not have the information about the probability, performing a weighted sum of the first data risk value and the second data risk value to determine the data risk. In this way, a weighted sum of the first data risk value and the second data risk value may be taken as the data risk, such that the information from both the sample dataset level and the population level is well balanced, and the data risk can be obtained accurately.

In some embodiments of the first aspect, processing at least the first data risk value and the second data risk value to determine the data risk comprises: responsive to a determination that the data recipient has the information about the probability, determining a third data risk value based on a re-identification probability of the target record in the sample dataset; performing a first weighted sum of the first data risk value and the second data risk value to determine a sum value; and performing a second weighted sum of the sum value and the third data risk value to determine the data risk. In this way, the re-identification probability of the target record may be used to determine the data risk based on the sample dataset. Thus, the determined data risk will be more customized for the data recipient, and the accuracy of the determined data risk will be improved.

In some embodiments of the first aspect, the method further comprises determining a weight of the first data risk value linearly based on a ratio between a size of the plurality of records in the sample dataset and a size of the population. In this way, a ratio between a size of the plurality of records in the sample dataset and a size of the population may be used for linearly determining a weight of the first data risk value, to avoid an imbalance between the first data risk value and the second data risk value.

In some embodiments of the first aspect, the method further comprises determining a weight of the first data risk value nonlinearly based on a ratio between a size of the plurality of records in the sample dataset and a size of the population. In this way, a ratio between a size of the plurality of records in the sample dataset and a size of the population may be used for nonlinearly determining a weight of the first data risk value, to avoid an imbalance between the first data risk value and the second data risk value.

In some embodiments of the first aspect, determining the weight of the first data risk value nonlinearly based on the ratio comprises: determining the weight of the first data risk value based on the ratio between the size of the plurality of records in the sample dataset and the size of the population by using a J shape distribution. In this way, a J shape distribution is used for determining a weight of the first data risk value, since the J shape distribution is one of widely used distributions, the application of J shape distribution will make the determination of the weight of the first data risk value simple.

In some embodiments of the first aspect, the method further comprises determining a weight of the third data risk value based on the probability. In this way, a probability that a target record is included in the sample dataset may be used as a weight of the third data risk value, and thus the weight is set based on the data recipient behavior (e.g., interests in certain data records). In this way, the data risk can be determined for the data recipient more accurately.

In some embodiments of the first aspect, determining the second data risk value comprises: determining the second data risk value by using one of the following manners: an entropy approach, a Bayesian approach, a hypothesis testing approach, or a synthetic estimator. In this way, a manner may be determined from various manners to determine the second data risk value, to improve a flexibility of the determination of the second data risk value.

In some embodiments of the first aspect, the method further comprises processing statistical data by using a predefined criterion to obtain the population. In this way, the statistical data can be used to determine the population, which may further be used to determine the second data risk value.

In some embodiments of the first aspect, there are multiple equivalence classes in the sample dataset, each equivalence class comprises multiple records which possess same attributes and share a same re-identification probability, and determining the first data risk value comprises: determining, for each equivalence class in the multiple equivalence classes, a re-identification probability shared by the multiple records in the each equivalence class; and determining the first data risk value by using one of the following manners: a maximum re-identification probability among the determined re-identification probabilities, an average of the determined re-identification probabilities, or a proportion of a quantity of equivalence classes, each of which has a re-identification probability higher than a threshold, to a quantity of the multiple equivalence classes. In this way, the first data risk value can be determined based on a re-identification probability of each record in the sample dataset by using one of multiple manners, to improve a flexibility of the determination of the first data risk value, thereby improving the accuracy of the determined data risk.

In some embodiments of the first aspect, each record possesses multiple attributes, and each attribute of the multiple attributes is associated with an indirect identification of the record. In this way, a direct identification of a record will not be included, thereby guaranteeing the security and privacy of the record.

In some embodiments of the first aspect, the method further comprises determining a context risk based on a maximum probability among the following probabilities: a probability of an insider attack of a data recipient, a probability that the sample dataset includes at least one record familiar to the data recipient, and a probability that the data recipient leaks the sample dataset; and processing a product of the data risk and the context risk to determine a re-identification risk. In this way, the context risk may be further determined, so as to determine a re-identification risk.

In a second aspect, the embodiments of the present disclosure provide an electronic device, comprising: at least one processor; and at least one memory having a plurality of instructions stored thereon, when executed by the at least one processor, cause the electronic device to perform the method of the first aspect.

In a third aspect, the embodiments of the present disclosure provide a computer readable medium having computer instructions stored thereon, the computer instructions, when executed a processor, causing the processor to perform the method of the first aspect.

It is to be understood that the Summary is not intended to identify key or essential features of embodiments of the present disclosure, nor is it intended to be used to limit the scope of the present disclosure. Other features of the present disclosure will become easily comprehensible through the description below.

BRIEF DESCRIPTION OF THE DRAWINGS

The details of one or more embodiments of the present disclosure are set forth in the accompanying drawings and the description below. Other features, aspects, and advantages of the disclosure will become apparent from the description, the drawings, and the claims, wherein:

FIG. 1A illustrates a block diagram of an example environment in which some embodiments of the present disclosure can be implemented;
FIG. 1B illustrates a table of an example sample dataset in accordance with aspects of the present disclosure;
FIG. 2 illustrates a flowchart of an example method for determining a data risk in accordance with aspects of the present disclosure;
FIG. 3 illustrates a flowchart of one example method for determining a data risk in accordance with aspects of the present disclosure;
FIG. 4 illustrates a flowchart of another example method for determining a data risk in accordance with aspects of the present disclosure; and
FIG. 5 illustrates a schematic block diagram of an example device suitable for implementing embodiments of the present disclosure.

Throughout the drawings, the same or similar reference numerals represent the same or similar element.

DETAILED DESCRIPTION OF EMBODIMENTS

The principles of the present disclosure will now be described with reference to some embodiments. It is to be understood that these embodiments are described only for the purpose of illustration and help those skilled in the art to understand and implement the present disclosure, without suggesting any limitation as to the scope of the disclosure. The disclosure described herein can be implemented in various manners other than the ones described below. In the following description and claims, unless defined otherwise, all technical and scientific terms used herein have the same meaning as commonly understood by one of ordinary skills in the art to which this disclosure belongs.

References in the present disclosure to "one embodiment," "an example embodiment," "an embodiment," "some embodiments," and the like indicate that the embodiment(s) described may include a particular feature, structure, or characteristic, but it is not necessary that every embodiment includes the particular feature, structure, or characteristic. Moreover, such phrases do not necessarily refer to the same embodiment(s). Further, when a particular feature, structure, or characteristic is described in connection with an embodiment, it is submitted that it is within the knowledge of one skilled in the art to affect such feature, structure, or characteristic in connection with other embodiments, whether or not explicitly described.

It shall be understood that although the terms "first" and "second" or the like may be used herein to describe various elements, these elements should not be limited by these terms. These terms are only used to distinguish one element from another element. For example, a first element could also be termed as a second element, and similarly, a second element could also be termed as a first element, without departing from the scope of embodiments. As used herein, the term "and/or" includes any and all combinations of one or more of the listed terms. In some examples, values, procedures, or apparatuses are referred to as "best," "lowest," "highest," "minimum," "maximum," or the like. It will be appreciated that such descriptions are intended to indicate that a selection among many used functional alternatives can be made, and such selections need not be better, smaller, higher, or otherwise preferable to other selections.

The terminology used herein is for the purpose of describing particular embodiments only and is not intended to be limiting to embodiments. As used herein, the singular forms "a," "an," and "the" are intended to include the plural forms as well, unless the context clearly indicates otherwise. It will be further understood that the terms "comprises," "comprising," "has," "having," "includes," and/or "including," when used herein, specify the presence of stated features, elements, components and/or the like, but do not preclude the presence or addition of one or more other features, elements, components and/or combinations thereof. For example, the term "includes" and its variants are to be read as open terms that mean "includes, but is not limited to." The term "based on" is to be read as "based at least in part on." The terms "one embodiment" and "an embodiment" are to be read as "at least one embodiment." The term "another embodiment" is to be read as "at least one other embodiment." The use of an expression such as "A and/or B" can mean either "only A" or "only B," or "both A and B." Other definitions, explicit and implicit, may be included below.

In the present disclosure, the term "determine" may be interchangeably used with one of the following: measure, estimate, calculate, obtain, acquire, etc. In some cases, the present disclosure does not limit this aspect.

In the present disclosure, a dataset may be a collection of data, e.g., in a form of table. A sample dataset may be a de-identified dataset resulting from an application of a de-identification approach, which is a common approach to protect personal privacy before disclosing the data for secondary purposes, such as research.

Data anonymization approaches may be used to determine whether the de-identification is suitable, for example, a re-identification risk may be obtained. Quantifying the re-identification risk is a critical step before disclosing the dataset to a data recipient, and the re-identification risk may be quantified as a percentage of records in a dataset that can be re-identified.

One of the data anonymization approaches involves using k-anonymity model. For examples, three risk metrics ($R_a$, $R_b$, and $R_c$) can be used for measuring the re-identification risk, which are specified in Table 1 below.

Table 1: re-identification risk metrics for k-anonymity

| Risk Metric | Interpretation |
|---|---|
| $R_a = \dfrac{1}{n} \sum_{j \in J} f_j \times I(\theta_j > \tau)$ | The proportion of records that have a re-identification probability higher than a threshold. |
| $R_b = \max_{j \in J}(\theta_j)$ | The maximum re-identification probability in the sample dataset among all re-cords. |
| $R_c = \dfrac{1}{n} \sum_{j \in J} f_j \theta_j$ | The proportion of records that can be correctly re-identified on average. |
| $n$ is the total number of records in the sample dataset. $J$ represents the set of equivalence classes in the sample dataset. $f_j$ is the size of equivalence class $j$ in the sample dataset. $\theta_j$ is a probability of a record in the equivalence class $j$ being correctly re-identified. $I(\cdot)$ is the indicator function (this returns 1 if the parameters are true and 0 otherwise). $\tau$ is the threshold which can be predefined based on risk preferences. | |

According to k-anonymity, $\theta_j$ may be calculated based on an attack type, which is one of prosecutor attack, journalist attack, or marketer attack. However, the prosecutor attack is based on an assumption that a data recipient knows a target record exists in the sample dataset, which means the determined risk does not apply to a general scenario. The journalist attack and the marketer attack depend on a database, specifically a database should be obtained and then be used to determine $\theta_j$. However, it is usually very costly to obtain a database, and it is even impossible to obtain the database sometime. In view of the above, it would be difficult to determine the risk.

To address these and other potential problems, embodiments of the present disclosure provide a solution for determining a data risk. In the solution, a first data risk value at the sample dataset level is determined, and a second data risk value at a population level is determined. Specifically, the first data risk value is determined based on a re-identification probability of each record in the sample dataset, and the second data risk value is determined based on statistical information of a population which includes the sample dataset. In addition, a data risk may be determined based on the first data risk value and the second data risk value. Embodiments of the present disclosure consider both a re-identification probability of each record in the sample dataset and statistical information of a population for determining the data risk. Therefore, the determined data risk will be more accurate, and thereby an accurate re-identification risk may be obtained accordingly.

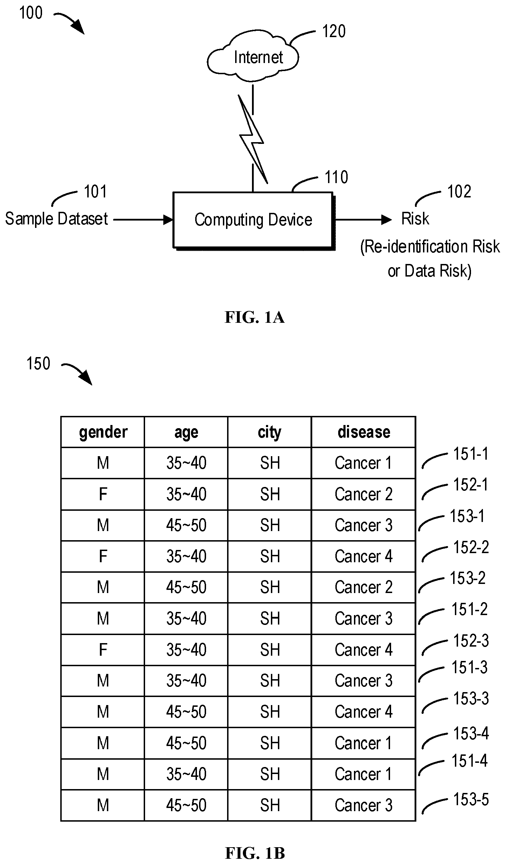

FIG. 1A illustrates a block diagram of an example environment 100 in which some embodiments of the present disclosure can be implemented. It would be appreciated that the environment 100 as shown therein is merely an example where embodiments of the present disclosure can be implemented, without suggesting limitations to the scope of the present disclosure. The embodiments of the present disclosure can also be applied to other systems or architecture.

As shown in FIG. 1A, the environment 100 may include a computing device 110. The computing device 110 may be any device having a computing capability. The computing device 110 may include, but is not limited to: a personal computer, a server computer, a handheld or laptop device, a mobile device (e.g., a mobile phone, a personal digital assistant (PDA), a media player or the like), a wearable device, a consumer electronic product, a small-scale computer, a large-scale computer, a distributed computing system, a cloud computing resource and the like.

The computing device 110 may have a networking capability, for example, the computing device 110 may connect to the internet 120 wirelessly or through a wire.

The computing device 110 may be configured to acquire a sample dataset 101, and determine a risk 102 of the sample dataset. The sample dataset 101 may be regarded as an input and the risk 102 may be regarded as an output. For example, the risk 102 may include a data risk. For example, the risk 102 may include a re-identification risk which is determined at least based on the data risk.

Exemplarily, the sample dataset 101 may be input by a user, or may be received from a storage device external to the computing device 110, or may be retrieved from a storage within the computing device 110, which is not limited in the present disclosure.

The sample dataset 101 may be a set of clinical data, a set of biomedical data, etc., and the sample dataset 101 includes a plurality of records. In some instances, each record in the sample dataset 101 may be related to a subject, for example, the data subject may be a person, such as a patient.

The sample dataset 101 may be a collection of personal data after a procedure of personal information de-identification by using a de-identification approach. For example, direct identifiers should be removed while performing the personal information de-identification, where a direct identifier refers to an attribute that alone enables unique identification of a subject. For example, the direct identifiers may include but are not limited to, name, passport number, social security number, phone number, Email address, etc.

For ease of description, it is assumed that the quantity of the plurality of records in the sample dataset 101 is $n$, which is a positive integer. In other words, there are $n$ records in the sample dataset 101. Alternatively, the size of the sample dataset 101 is $n$. Each record in the sample dataset 101 may possess multiple attributes. Each of the multiple attributes of a record in the sample dataset 101 is an indirect identifier, i.e., not a direct identifier. For example, an attribute of the record may be one of quasi-identifiers, where a quasi-identifier refers to an attribute that alone cannot be used to identify a subject. For example, the quasi-identifiers may include, but are not limited to, age, gender, nationality, career, etc.

There may be one or more equivalence classes in the sample dataset 101. For example, a set of equivalence classes in the sample dataset 101 may be represented as $J$, and the quantity of the equivalence classes may be represented as $|J|$. An equivalence class may include one or more records with the same quasi-identifiers. For an equivalence class (such as $j \in J$), the quantity of the one or more records in the equivalence class may refer to a size of the equivalence class, which may be represented as $f_j$.

All records in a same equivalence class have a same re-identification probability, for example, a re-identification probability for each record in the equivalence class $j$ is represented as $\theta_j$, which can be determined based on equation (1):

$$\theta_j = \frac{1}{f_j} \tag{1}$$

In some implementations, the sample dataset 101 may be in a form of table. FIG. 1B illustrates a table 150 of an example sample dataset in accordance with aspects of the present disclosure. As shown in FIG. 1B, the table 150 may be associated with a matrix structure having multiple rows and multiple columns. A row may be a record in the sample dataset 101. A column may represent an attribute of a record, e.g., a quasi-identifier or other information of the record (such as "disease" in FIG. 1B). However, it is to be understood that a record may be stored as a column in some other cases.

In FIG. 1B, there are 12 records, that is $n$ = 12. Each record has multiple quasi-identifiers, including gender, age, and city. Multiple equivalence classes can be determined, specifically, multiple records with the same quasi-identifiers belong to a same equivalence class. Refer to FIG. 1B, records 151-1, 151-2, 151-3, and 151-4 possess the same quasi-identifiers: "gender: M," "age: 35-40", and "city: SH," and thus belong to a same equivalence class, i.e., equivalence class 1. Refer to FIG. 1B, records 152-1, 152-2, and 152-3 possess the same quasi-identifiers: "gender: F", "age: 35-40", and "city: SH," and thus belong to a same equivalence class, i.e., equivalence class 2. Refer to FIG. 1B, records 153-1, 153-2, 153-3, 153-4, and 153-5 possess the same quasi-identifiers: "gender: M," "age: 45-50", and "city: SH," and thus belong to a same equivalence class, i.e., equivalence class 3. Hence, three equivalence classes, i.e., equivalence class 1, equivalence class 2, and equivalence class 3, are determined accordingly, that is $J$ = {equivalence class 1, equivalence class 2, equivalence class 3} and $|J|$ = 3. Specifically, equivalence class 1 includes records 151-1, 151-2, 151-3, and 151-4, equivalence class 2 includes records 152-1, 152-2, and 152-3, and equivalence class 3 includes records 153-1, 153-2, 153-3, 153-4, and 153-5. A size of equivalence class 1 is 4, a size of equivalence class 2 is 3, and a size of equivalence class 3 is 5. Accordingly, the re-identification probabilities ($\theta_j$) for each record in equivalence class 1, equivalence class 2, and equivalence class 3 are 1/4, 1/3, and 1/5, respectively.

It should be noted that the table 150 also includes a column "disease" which is not an identifier (i.e., neither direct identifier nor quasi-identifier). It is to be understood that the table 150 shown in FIG. 1B is only for illustration without any limitation, for example, in some other cases, more or less quasi-identifiers may be included, more or less records may be included, sizes of different equivalence classes may be the same, the present disclosure does not limit this aspect. It is to be appreciated that although table 150 is shown as a form of the sample dataset 101, in some other examples, the sample dataset 101 may be in another form other than the table, the present disclosure does not limit this aspect.

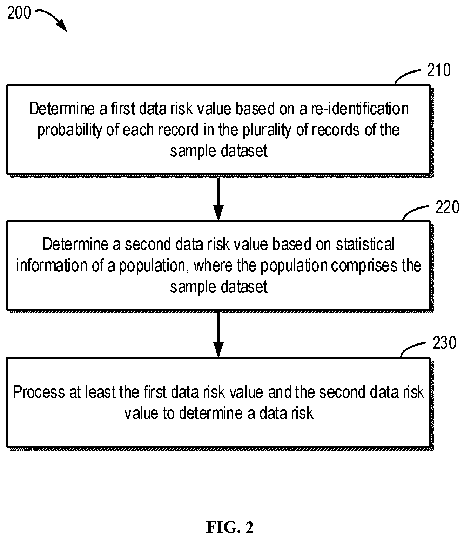

The present disclosure provides a solution for determining a data risk, details of which will be discussed with reference to FIGS. 2-4 below.

Reference is now made to FIG. 2, which illustrates an example method 200 for determining a data risk in accordance with aspects of the present disclosure. The method 200 may be executed by an electronic device such as the computing device 110 as shown in FIG. 1A. The following embodiments will be described with reference to the computing device 110, but it would be appreciated that the present disclosure does not limit.

At step 210, the computing device 110 determines a first data risk value based on a re-identification probability of each record in the plurality of records of the sample dataset 101. In some examples, the first data risk value may be also called as a data risk at sample dataset level.

In some implementations, for each equivalence class in the sample dataset 101, the computing device 110 may determine a re-identification probability ($\theta_j$) for each record in the equivalence class. For example, a re-identification probability for each record in the equivalence class may be determined according to equation (1) described above. Since all records in a same equivalence class share a same re-identification probability, the re-identification probability for each equivalence class of $|J|$ equivalence classes is also determined. Further, the computing device 110 may determine the first data risk value based on the determined $|J|$ re-identification probabilities for the $|J|$ equivalence classes.

In some implementations, the first data risk value may be represented as $R_s$, which may be determined according to one of equations (2)-(4) below, where $\theta_j$ is based on equation (1) above.

$$R_s = \frac{1}{|J|}\sum_{j\in J} I(\theta_j > \tau) \tag{2}$$

$$R_s = \max_{j\in J} \theta_j \tag{3}$$

$$R_s = \frac{1}{|J|}\sum_{j\in J} \theta_j \tag{4}$$

Specifically, $R_s$ in equation (2) represents a proportion of a quantity of equivalence classes, each of which has a re-identification probability higher than a threshold, to a quantity of all equivalence classes in the sample dataset 101. For example, the threshold ($\tau$) may be a predefined value ranging from 0 to 1, such as 0.05, 0.2, 0.3, or another value. With reference to FIG. 1B, if the threshold is 0.3, then $R_s = 1/3$ according to equation (2).

Specifically, $R_s$ in equation (3) represents a maximum re-identification probability among the determined $|J|$ re-identification probabilities. With reference to FIG. 1B, $R_s = 1/3$ according to equation (3).

Specifically, $R_s$ in equation (4) represents an average of the determined $|J|$ re-identification probabilities. With reference to FIG. 1B, $R_s = 47/180$ according to equation (4).

At step 220, the computing device 110 determines a second data risk value based on statistical information of a population, where the population comprises the sample dataset 101. The population may include all records in the sample dataset. In some examples, the second data risk value may also be called as a data risk value at a population level. In some implementations, the computing device 110 may obtain statistical data, and further process the statistical data using a predefined criterion so as to obtain the population.

In some example embodiments, the statistical data may be referred to as a raw population or a prevalent population. For example, the statistical data may be obtained from an available data base. For example, the available data base may be established based on a census or study results, and the computing device 110 can inquire the available data base from the internet 120.

For each equivalence class in the sample dataset 101, a corresponding population may be determined based on the statistical data and a distribution of each quasi-identifier of the equivalence class. Specifically, a corresponding population for the equivalence class may refer to a part of the population having the same quasi-identifiers as the records in the equivalence class. In addition, the population may be further determined based on a sum of all determined corresponding populations.

In some examples, for each equivalence class, the distribution of each quasi-identifier of the equivalence class may be uniquely determined. For example, if the statistical data is represented as $P_0$, then a corresponding population of the equivalence class $j$ may be represented as $F_j$ and may be determined based on equation (5) below:

$$\min_{j\in J}(F_j) > \prod_{q\in Q} dist(q) \times P_0 \tag{5}$$

In equation (5), *Q* refers to a set of quasi-identifiers of the equivalence class *j*, *dist*(*q*) refers to a distribution of a quasi-identifier *q* with $q \in Q$.

In some other examples, for each equivalence class, the distribution of each quasi-identifier of the equivalence class may not be uniquely determined. For example, there may be multiple sources disclosing the distribution of each quasi-identifier of the equivalence class but with different values. In this case, a minimum distribution of each quasi-identifier of the equivalence class may be used for determining the corresponding population. For example, equation (6) may be used rather than equation (5):

$$\min_{j \in J}(F_j) > \prod_{q \in Q} \min_{b \in B}(dist(q)) \times P_0 \qquad (6)$$

In equation (6), *B* refers to a set of multiple sources, *b* refers to one source, and *dist*(*q*) refers to a distribution of a quasi-identifier *q* with $q \in Q$ disclosed by the source *b*.

In some embodiments, the corresponding population of an equivalence class, which may be represented as $F_j$, can be determined to be equal to the right part of the equation (5) or (6). In some other embodiment, the corresponding population of an equivalence class, which may be represented as $F_j$, can be determined to be equal to the right part of the equation (5) or (6) multiplying a factor. For example, the factor is a number larger than 1.

In addition, the population may be determined as a sum of all determined corresponding populations ($F_j$), for ease of description, the population may be represented as *N*. It is to be understood that $N > n$, e.g., the sample dataset is a subset of the population.

In some implementations, the second data risk value may be determined by using one of the following manners: an entropy approach, a Bayesian approach, a hypothesis testing approach, or a synthetic estimator.

In some examples, the entropy approach measures the degree of uncertainty in the outcome of a random variable. The reciprocal measure of entropy is information content, which quantifies the expected contribution of a new piece of data in reducing the entropy level. Therefore, information content can be used to capture the average usefulness of quasi-identifiers for identity tracing. For example, entropies (can be calculated with the knowledge of all the distributions at population level) of each quasi-identifier may be used for determining the second data risk value.

In some examples, the Bayesian approach provides a population estimate with an associated uncertainty. It starts with an initial estimation based on published statistical data (such as the available prevalence population published), and then a projection needs to be made, e.g., a cohort component method of population projection (CCMPP). Finally, a joint posterior distribution of the input parameters can serve as an inference for the parameters of the population. Specifically, theoretical distributions at population level (from external/public reports, studies) and the distributions from the sample data may be used together to determine the second data risk value.

In some examples, the hypothesis testing approach usually assumes that a size of an equivalence class in the sample dataset follows some distribution pattern, such as Poisson distribution. Then, a hypothesis testing approach may be used for determining a boundary of the population level size for each equivalence class. Specifically, each size of an equivalence class in the sample dataset and an acceptable level of error (alpha) may be used for determining the second data risk value.

In some examples, the synthetic estimator creates a synthetic population level data based on the sample dataset, and then randomly selects data from the generated synthetic population level data to calculate the re-identification risk. Copulas (e.g., Gaussian copula, d-vine copula) usually are used to model the inter-correlation between random variables in the process of creating synthetic population level dataset.

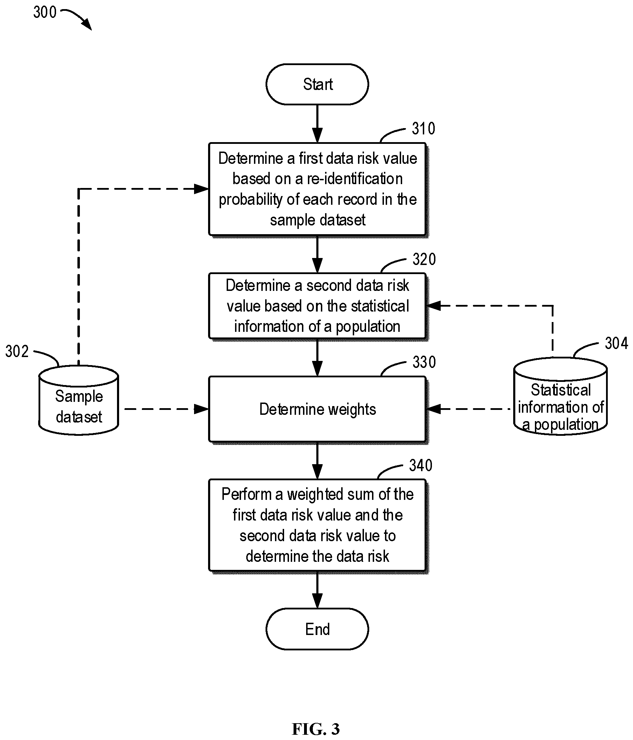

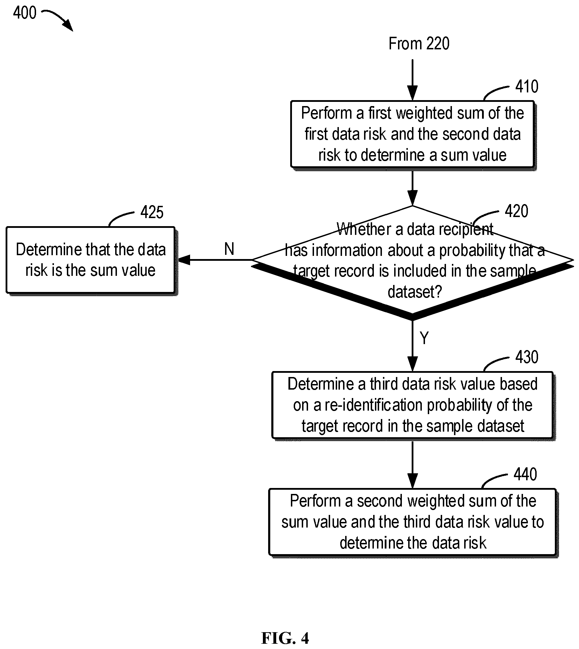

At step 230, the computing device 110 processes at least the first data risk value and the second data risk value to determine a data risk. In some implementations, the data risk may be determined based on a weighted sum of the first data risk value and the second data risk value, details of which may refer to FIG. 3. In some other implementations, whether a data recipient of the sample dataset 101 has information about a probability that a target record is included in the sample dataset may be considered while determining the data risk, details of which may refer to FIG. 4.

FIG. 3 illustrates an example method 300 for determining a data risk in accordance with aspects of the present disclosure. At step 310, the first data risk value may be determined based on a re-identification probability of each record in the sample dataset 302, details of the step 310 may refer to step 210 in FIG. 2, and thus will not be repeated herein. At step 320, the second data risk value may be determined based on the statistical information of a population 304, details of the step 320 may refer to step 210 in FIG. 2, and thus will not be repeated herein.

At step 330, weights are determined, specifically, the weights are used for the weighted sum of the first data risk value and the second data risk value. For example, a weight of the first data risk value may be represented as $W_s$, and a weight of the second data risk value may be represented as $W_p$, with $W_s + W_p = 1$.

A ratio between a size of the plurality of records in the sample dataset and a size of the population, i.e., *n/N*, may be determined, and be further used to determine the weights $W_s$ and $W_p$. In some examples, the ratio may also be called a sample fraction denoted by $fra_s$.

In some examples, the weight of the first data risk value ($W_s$) may be linearly based on the ratio. For example, the following equation (7) may be used for determining the weight of the first data risk value ($W_s$):

$$W_s = \alpha \frac{n}{N} + (1 - \alpha) \tag{7}$$

In equation (7), $\alpha$ is a predefined positive value not larger than 1. In other words, $\alpha$ is a value ranging from 0 to 1.

In some other examples, the weight of the first data risk value ($W_s$) may be nonlinearly based on the ratio. For example, the following equation (8) may be used for determining the weight of the first data risk value ($W_s$):

$$W_s = \alpha \frac{n}{N} \left(2 - \frac{n}{N}\right)^\gamma + (1 - \alpha) \frac{n}{N} \tag{8}$$

In equation (8), $\alpha$ is a predefined positive value not larger than 1 (i.e., a value ranging from 0 to 1), and $\gamma$ is a value in a range of 0~1 (i.e., a value ranging from 0 to 1).

In some instances, the equation (8) may be determined by using the J shape distribution. For example, the J shape distribution is one of widely used distributions in many fields of research. Specifically, the J shape distribution may be an expression with parameters $\beta$ and $x$, if the parameters are set as $\beta = 1$ and $x = n/N$, then a cumulative distribution function (CDF) of the J Shape distribution may be obtained and be used as equation (8). For example, $\gamma$ may be one of 0.5, 0.75, or 0.95 determined by using the J shape distribution. However, it is to be noted that the value of $\gamma$ is not limited in the present disclosure.

In addition, the weight of the second data risk value ($W_p$) may be determined based on equation (9), after the weight of the first data risk value ($W_s$) is determined.

$$W_p = 1 - W_s \tag{9}$$

It should be appreciated that although the above embodiments of step 330 disclose that the weight of the first data risk value is determined before determining the weight of the second data risk value, the present disclosure does not limit this aspect, for example, the weight of the second data risk value may be determined firstly in some other embodiments, details of which will not be presented herein for brevity.

At step 340, the weighted sum of the first data risk value and the second data risk value is performed to determine the data risk. Specifically, the data risk may be determined based on equation (10) below:

$$R = W_s \times R_s + W_p \times R_p \tag{10}$$

As such, the data risk may be determined based on a weighted sum of the first data risk value and the second data risk value, where the weight of the first data risk value is determined according to equation (7) or (8), which is based on the ratio $n/N$, and the weight of the second data risk value is determined according to equation (9). It can be seen that, for a smaller ratio, less information of the first data risk value will be leveraged; and for a larger ratio, more information of the first data risk value will be leveraged. Therefore, data risk values based on both a re-identification probability of each record in the sample dataset and statistical information of a population are considered, helping to avoid a large estimation variance.

FIG. 4 illustrates an example method 400 for determining a data risk in accordance with aspects of the present disclosure. It is assumed that the method 400 is performed after the step 220 in FIG. 2 for brevity. In other words, the step 410 is performed after determining the first data risk value and the second data risk value.

At step 410, a first weighted sum of the first data risk value and the second data risk value is performed to determine a sum value. In some implementations, the first weighted sum may be performed in a similar way as the weighted sum described with reference to FIG. 3. For example, if the sum value is represented as $R_1$, then it equals to $W_s \times R_s + W_p \times R_p$.

At step 420, whether a data recipient of the sample dataset has information about a probability that a target record is included in the sample dataset may be determined.

In the present disclosure, the data risk for a subject is to be determined. The subject may be included in the sample dataset in some cases or maybe not included in the sample dataset. Further, a probability that a subject is included in the sample dataset may be considered. For example, the subject may be an individual (e.g., a target individual) the data recipient is interested in, or a record the data recipient intends to study. In the present disclosure, the subject which the data recipient is interested in is called a target record for ease of description.

If the data recipient does not have the information about a probability that a target record is included in the sample dataset, the step 425 is further performed. At step 425, the sum value determined at step 420 is determined as the data risk. That is, the following equation (11) is applied:

$$R = R_1 = W_s \times R_s + W_p \times R_p \qquad\qquad (11)$$

In this case, the data risk determined at step 425 is the same as that determined at step 330 in FIG. 3.

Otherwise, if it is determined that the data recipient has the information about a probability that a target record is included in the sample dataset at 420, the step 430 is further performed. For example, the probability that the target record is included in the sample dataset is denoted as $P_T$ (a value ranging from 0 to 1), which is known by the data recipient.

In the present disclosure, the target record may be an individual the data recipient is interested in, or a record the data recipient intends to study. For example, the target record may be a target individual for the data recipient.

At step 430, a third data risk value is determined based on a re-identification probability of the target record in the sample dataset. The third data risk value is associated with the target record and may be represented as $R_T$. Specifically, the third data risk value may equal to a re-identification probability of the target record in the sample dataset. For example, $\theta_j$ for the equivalence class, which include the target record may be determined as the third data risk value.

At step 440, a second weighted sum of the sum value and the third data risk value is performed to determine the data risk. Specifically, a weight of the third data risk value may equal to $P_T$, i.e., the probability that the target record is included in the sample dataset, and the following equation (12) may be used for determining the data risk:

$$R = P_T \times R_T + (1 - P_T) \times R_1 = P_T \times R_T + (1 - P_T) \times \left(W_s \times R_s + W_p \times R_p\right) \quad (12)$$

It is to be appreciated that the steps in FIGS. 2-4 are shown only for illustration without any limitation. In some cases, one or more steps may be omitted, one or more additional steps may be added, one or more steps may be modified, some steps may be combined, or the order of steps may be changed. For an example, steps 330 and 340 can be combined into one step. For an example, step 410 may be performed after step 420, e.g. between step 420 and step 425 or between step 420 and step 430.

According to embodiments with reference to FIGS. 2-4, a data risk is determined. In some implementations, the computing device 110 may determine a context risk, and in addition determine a re-identification risk based on the data risk and the context risk.

The context risk may be determined based on a maximum probability of the following three probabilities: a probability of an insider attack of a data recipient, a probability that the sample dataset includes at least one record familiar to the data recipient, and a probability that the data recipient leaks the sample dataset.

For example, an insider (e.g., an employee) of the data recipient may launch an attack, e.g., releasing or disclosing one or more records in the sample dataset without a permission of the data recipient. For example, the probability of an insider attack of the data recipient may be determined based on a security level of the data recipient of the sample dataset and a motivation level for using the sample dataset by the data recipient.

For example, the data recipient has acquaintances, and at least one record in the sample dataset may belong to the acquaintances. In this case, the at least one record may be familiar to the data recipient. For example, the probability that the sample dataset includes at least one record familiar to the data recipient may be determined based on a percentage of all records with the attributes in the sample dataset and the number of acquaintances of the data recipient.

For example, the probability that the data recipient leaks the sample dataset may be determined based on a capability for data security and privacy control of the data recipient.

In addition, the re-identification risk may be determined by multiplying the data risk and the context risk. For example, if the context risk is denoted as $R_c$, then the re-identification risk may be represented as $R \times R_c$.

As such, the re-identification risk can be quantified as a product of the data risk and the context risk. The quantification can be used to evaluate a result of the de-identification process of personal data, thus ensuing the security of personal data.

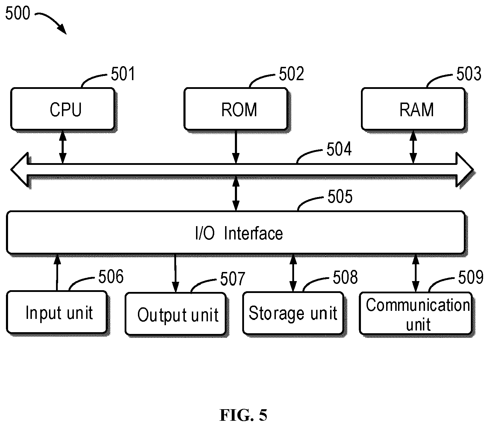

FIG. 5 illustrates a schematic block diagram of an example device 500 suitable for implementing embodiments of the present disclosure. The computing device 110 in FIG. 1A may be implemented by the device 500. As shown in FIG. 5, the device 500 comprises a central process unit (CPU) 501, which can execute various suitable actions and processing based on the computer program instructions stored in a read-only memory (ROM) 502 or computer program instructions loaded in the random-access memory (RAM) 503 from a storage unit 508. The RAM 503 can also store all kinds of programs and data required by the operation of the device 500. CPU 501, ROM 502, and RAM 503 are connected to each other via a bus 504. The input/output (I/O) interface 505 is also connected to the bus 504.

A plurality of components in the device 500 is connected to the I/O interface 505, including: an input unit 506, such as a keyboard, a mouse, and the like; an output unit 507, e.g., various kinds of display and loudspeakers etc.; a storage unit 508, such as a disk and an optical disk etc.; and a communication unit 509, such as a network card, a modem, a wireless transceiver and the like. The communication unit 509 allows the device 500 to exchange information/data with other devices via the computer network, such as Internet, and/or various telecommunication networks.

The above-described procedure and processing, such as methods 200-400, can be executed by the CPU 501. For example, in some embodiments, the methods 200-400 can be implemented as a computer software program tangibly included in the machine-readable medium, e.g., storage unit 508. In some embodiments, the computer program can be partially or fully loaded and/or mounted to the device 500 via ROM 502 and/or communication unit 509. When the computer program is loaded to RAM 503 and executed by the CPU 501, one or more actions of the above describe methods 200-400 can be implemented.

The present disclosure can be a method, an electric device, a computer readable medium, and/or a computer program product. The computer program product can include a computer-readable storage medium, on which the computer-readable program instructions for executing various aspects of the present disclosure are loaded.

The functions described herein may be implemented in hardware, software executed by a processor, firmware, or any combination thereof. If implemented in software executed by a processor, the functions may be stored on or transmitted over as one or more instructions or code on a computer-readable medium. Other examples and implementations are within the scope of the disclosure and appended claims. For example, due to the nature of software, functions described herein may be implemented using software executed by a processor, hardware, firmware, hardwiring, or combinations of any of these. Features implementing functions may also be physically located at various positions, including being distributed such that portions of functions are implemented at different physical locations.

The computer-readable storage medium can be a tangible apparatus that maintains and stores instructions utilized by the instruction executing apparatuses. The computer-readable storage medium can be, but is not limited to, such as an electrical storage device, a magnetic storage device, an optical storage device, an electromagnetic storage device, a semiconductor storage device, or any appropriate combinations of the above. More concrete examples of the computer-readable storage medium (non-exhaustive list) include: a portable computer disk, a hard disk, a RAM, a ROM, an erasable programmable read-only memory (EPROM or flash), a static random-access memory (SRAM), a portable compact disk read-only memory (CD-ROM), a digital versatile disk (DVD), a memory stick, a floppy disk, mechanical coding devices, a punched card stored with instructions thereon, or a projection in a slot, and any appropriate combinations of the above. The computer-readable storage medium utilized here is not interpreted as transient signals per se, such as radio waves or freely propagated electromagnetic waves, electromagnetic waves propagated via waveguide or other transmission media (such as optical pulses via fiber-optic cables), or electric signals propagated via electric wires.

The described computer-readable program instruction can be downloaded from the computer-readable storage medium to each computing/processing device, or to an external computer or external storage via Internet, local area network, wide area network and/or wireless network. The network can comprise copper-transmitted cable, optical fiber transmission, wireless transmission, router, firewall, switch, network gate computer and/or edge server. The network adapter card or network interface in each computing/processing device receives computer-readable program instructions from the network and forwards the computer-readable program instructions for storage in the computer-readable storage medium of each computing/processing device.

The computer program instructions for executing operations of the present disclosure can be assembly instructions, instructions of instruction set architecture (ISA), machine instructions, machine-related instructions, micro codes, firmware instructions, state setting data, or source codes or target codes written in any combinations of one or more programming languages, wherein the programming languages comprise object-oriented programming languages, e.g., Smalltalk, C++ and so on, and traditional procedural programming languages, such as "C" language or similar programming languages. The computer-readable program instructions can be implemented fully on the user computer, partially on the user computer, as an independent software package, partially on the user computer and partially on the remote computer, or completely on the remote computer or server. In the case where remote computer is involved, the remote computer can be connected to the user computer via any type of networks, including local area network (LAN) and wide area network (WAN), or to the external computer (e.g., connected via Internet using the Internet service provider). In some embodiments, state information of the computer-readable program instructions is used to customize an electronic circuit, e.g., programmable logic circuit, field programmable gate array (FPGA), or programmable logic array (PLA). The electronic circuit can execute computer-readable program instructions to implement various aspects of the present disclosure.

Various aspects of the present disclosure are described here with reference to flow chart and/or block diagram of method, apparatus (system), and computer program products according to embodiments of the present disclosure. It should be understood that each block of the flow chart and/or block diagram and the combination of various blocks in the flow chart and/or block diagram can be implemented by computer-readable program instructions.

The computer-readable program instructions can be provided to the processing unit of general-purpose computer, dedicated computer, or other programmable data processing apparatuses to manufacture a machine, such that the instructions that, when executed by the processing unit of the computer or other programmable data processing apparatuses, generate an apparatus for implementing functions/actions stipulated in one or more blocks in the flow chart and/or block diagram. The computer-readable program instructions can also be stored in the computer-readable storage medium and cause the computer, programmable data processing apparatus and/or other devices to work in a particular manner, such that the computer-readable medium stored with instructions comprises an article of manufacture, including instructions for implementing various aspects of the functions/actions stipulated in one or more blocks of the flow chart and/or block diagram.

The computer-readable program instructions can also be loaded into computer, other programmable data processing apparatuses, or other devices, so as to execute a series of operation steps on the computer, other programmable data processing apparatuses, or other devices to generate a computer-implemented procedure. Therefore, the instructions executed on the computer, other programmable data processing apparatuses, or other devices implement functions/actions stipulated in one or more blocks of the flow chart and/or block diagram.

The flow chart and block diagram in the drawings illustrate system architecture, functions, and operations that may be implemented by system, method, and computer program product according to multiple implementations of the present disclosure. In this regard, each block in the flow chart or block diagram can represent a module, a part of program segment or code, wherein the module and the part of program segment or code include one or more executable instructions for performing stipulated logic functions. In some alternative implementations, it should be noted that the functions indicated in the block can also take place in an order different from the one indicated in the drawings. For example, two successive blocks can be in fact executed in parallel or sometimes in a reverse order dependent on the involved functions. It should also be noted that each block in the block diagram and/or flow chart and combinations of the blocks in the block diagram and/or flow chart can be implemented by a hardware-based system exclusive for executing stipulated functions or actions, or by a combination of dedicated hardware and computer instructions.

Various embodiments of the present disclosure have been described above, and the above description is only exemplary rather than exhaustive and is not limited to the embodiments of the present disclosure. Many modifications and alterations, without deviating from the scope and spirit of the explained various embodiments, are obvious for those skilled in the art. The selection of terms in the text aims to best explain principles and actual applications of each embodiment and technical improvements made in the market by each embodiment, or enable those ordinary skilled in the art to understand embodiments of the present disclosure.

## Claims

1.   A method for determining a data risk, the method comprises:

determining a first data risk value based on a re-identification probability of each record in the plurality of records in a sample dataset;
determining a second data risk value based on statistical information of a population, wherein the population comprises the sample dataset; and
processing at least the first data risk value and the second data risk value to determine the data risk.

2.   The method of claim 1, further comprising:
determining whether or not a data recipient of the sample dataset has information about a probability that a target record is included in the sample dataset.

3.   The method of claim 2, wherein processing at least the first data risk value and the second data risk value to determine the data risk comprises:
responsive to a determination that the data recipient does not have the information about the probability, performing a weighted sum of the first data risk value and the second data risk value to determine the data risk.

4.   The method of claim 2, wherein processing at least the first data risk value and the second data risk value to determine the data risk comprises:

responsive to a determination that the data recipient has the information about the probability, determining a third data risk value based on a re-identification probability of the target record in the sample dataset;
performing a first weighted sum of the first data risk value and the second data risk value to determine a sum value; and
performing a second weighted sum of the sum value and the third data risk value to determine the data risk.

5.   The method of claim 3 or 4, further comprising:

determining a weight of the first data risk value linearly based on a ratio between a size of the plurality of records in the sample dataset and a size of the population.

6. The method of claim 3 or 4, further comprising:
determining a weight of the first data risk value nonlinearly based on a ratio between a size of the plurality of records in the sample dataset and a size of the population.

7. The method of claim 6, wherein determining the weight of the first data risk value nonlinearly based on the ratio comprises:
determining the weight of the first data risk value based on the ratio between the size of the plurality of records in the sample dataset and the size of the population by using a J shape distribution.

8. The method of claim 4, further comprising:
determining a weight of the third data risk value based on the probability.

9. The method of claim 1, wherein determining the second data risk value comprises:
determining the second data risk value by using one of the following manners: an entropy approach, a Bayesian approach, a hypothesis testing approach, or a synthetic estimator.

10. The method of claim 1, further comprising:
processing statistical data by using a predefined criterion to obtain the population.

11. The method of claim 1, wherein there are multiple equivalence classes in the sample dataset, each equivalence class comprises multiple records which possess same attributes and share a same re-identification probability, and wherein determining the first data risk value comprises:

   determining, for each equivalence class in the multiple equivalence classes, a re-identification probability shared by the multiple records in the each equivalence class; and
   determining the first data risk value by using one of the following manners:

   a maximum re-identification probability among the determined re-identification probabilities,
   an average of the determined re-identification probabilities, or
   a proportion of a quantity of equivalence classes, each of which has a re-identification probability higher than a threshold, to a quantity of the multiple equivalence classes.

12. The method of claim 1, wherein each record possesses multiple attributes, and each attribute of the multiple attributes is associated with an indirect identification of the record.

13. The method of claim 1, further comprising:

   determining a context risk based on a maximum probability among the following probabilities: a probability of an insider attack of a data recipient, a probability that the sample dataset includes at least one record familiar to the data recipient, and a probability that the data recipient leaks the sample dataset; and
   processing a product of the data risk and the context risk to determine a re-identification risk.

14. An electronic device, comprising:

   at least one processor; and
   at least one memory having a plurality of instructions stored thereon, when executed by the at least one processor, cause the electronic device to determine a data risk, and the electronic device is caused to:

   determine a first data risk value based on a re-identification probability of each record in the plurality of records in a sample dataset;
   determine a second data risk value based on statistical information of a population, wherein the population comprises the sample dataset; and
   process at least the first data risk value and the second data risk value to determine the data risk.

15. A computer program product having computer instructions thereon, the computer instructions, when executed by a computer or a processor, causing the computer or the processor to perform the method according to any of claims 1-13.

100

120
Internet

101
Sample Dataset ——→

110
Computing Device

102
Risk
(Re-identification Risk
or Data Risk)

**FIG. 1A**

150

| gender | age | city | disease | |
|--------|-------|------|----------|---------|
| M | 35~40 | SH | Cancer 1 | 151-1 |
| F | 35~40 | SH | Cancer 2 | 152-1 |
| M | 45~50 | SH | Cancer 3 | 153-1 |
| F | 35~40 | SH | Cancer 4 | 152-2 |
| M | 45~50 | SH | Cancer 2 | 153-2 |
| M | 35~40 | SH | Cancer 3 | 151-2 |
| F | 35~40 | SH | Cancer 4 | 152-3 |
| M | 35~40 | SH | Cancer 3 | 151-3 |
| M | 45~50 | SH | Cancer 4 | 153-3 |
| M | 45~50 | SH | Cancer 1 | 153-4 |
| M | 35~40 | SH | Cancer 1 | 151-4 |
| M | 45~50 | SH | Cancer 3 | 153-5 |

**FIG. 1B**

200

210

Determine a first data risk value based on a re-identification probability of each record in the plurality of records of the sample dataset

220

Determine a second data risk value based on statistical information of a population, where the population comprises the sample dataset

230

Process at least the first data risk value and the second data risk value to determine a data risk

FIG. 2

300

Start

310

Determine a first data risk value based on a re-identification probability of each record in the sample dataset

320

Determine a second data risk value based on the statistical information of a population

302

Sample dataset

330

Determine weights

304

Statistical information of a population

340

Perform a weighted sum of the first data risk value and the second data risk value to determine the data risk

End

**FIG. 3**

400

From 220

Perform a first weighted sum of the
first data risk and the second data
risk to determine a sum value
410

Whether a data recipient
has information about a probability that a
target record is included in the sample
dataset?
420

N

Determine that the data
risk is the sum value
425

Y

Determine a third data risk value based
on a re-identification probability of the
target record in the sample dataset
430

Perform a second weighted sum of the
sum value and the third data risk value to
determine the data risk
440

**FIG. 4**

500

501
CPU

502
ROM

503
RAM

504

505
I/O Interface

506
Input unit

507
Output unit

508
Storage unit

509
Communication unit

**FIG. 5**

Europäisches
Patentamt

European
Patent Office

Office européen
des brevets

# EUROPEAN SEARCH REPORT

**Application Number**

EP 24 16 4025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/307104 A1 (KORTE STEPHEN [CA] ET AL) 28 September 2023 (2023-09-28) * paragraph [0029] * * paragraph [0031] - paragraph [0032] * * paragraph [0043] * | 1-15 | INV. G06F21/62 |
| A | US 2023/148326 A1 (PAPEL OSCAR [US] ET AL) 11 May 2023 (2023-05-11) * paragraphs [0070], [0104] * | 3,13 | |
| A | US 2022/300651 A1 (MONDAL SUTAPA [IN] ET AL) 22 September 2022 (2022-09-22) * paragraph [0060] - paragraph [0061]; figure 5a * | 1-15 | |
| A | US 2021/049282 A1 (DI VALENTINO DAVID NICHOLAS MAURICE [CA] ET AL) 18 February 2021 (2021-02-18) * paragraph [0044] - paragraph [0059] * | 1-15 | |
| A | Anonymous: "De-identification 301", , 30 November 2020 (2020-11-30), pages 1-10, XP093189649, Internet archive Retrieved from the Internet: URL:https://web.archive.org/web/2020113011 3553if_/https://privacy-analytics.com/wp-c ontent/uploads/dlm_uploads/2020/06/De-Id-3 01-White-Paper.pdf [retrieved on 2024-07-25] * the whole document * | 1-15 | **TECHNICAL FIELDS SEARCHED (IPC)** G06F G16H |
| A | US 2022/050917 A1 (JIANG YANGDI [CA] ET AL) 17 February 2022 (2022-02-17) * title * | 9 | |

The present search report has been drawn up for all claims

| Place of search | Date of completion of the search | Examiner |
|---|---|---|
| Munich | 26 July 2024 | Alyaz, Eymen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 4025

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2024

| Patent document cited in search report | Publication date | Patent family member(s) | | Publication date |
|---|---|---|---|---|
| US 2023307104 A1 | 28-09-2023 | CA | 2913561 A1 | 27-05-2016 |
| | | US | 2016155061 A1 | 02-06-2016 |
| | | US | 2022115101 A1 | 14-04-2022 |
| | | US | 2023307104 A1 | 28-09-2023 |
| US 2023148326 A1 | 11-05-2023 | US | 2023148326 A1 | 11-05-2023 |
| | | WO | 2023081919 A1 | 11-05-2023 |
| US 2022300651 A1 | 22-09-2022 | EP | 4060543 A1 | 21-09-2022 |
| | | US | 2022300651 A1 | 22-09-2022 |
| US 2021049282 A1 | 18-02-2021 | CA | 3089835 A1 | 12-02-2021 |
| | | EP | 3779757 A1 | 17-02-2021 |
| | | US | 2021049282 A1 | 18-02-2021 |
| US 2022050917 A1 | 17-02-2022 | EP | 3955151 A1 | 16-02-2022 |
| | | US | 2022050917 A1 | 17-02-2022 |

EPO FORM P0459

For more details about this annex : see Official Journal of the European Patent Office, No. 12/82